# EUROPEAN PATENT APPLICATION

(11) **EP 2 813 852 A2**
(43) Date of publication of application: **17.12.2014**
(21) Application number: 14171891.6
(22) Date of filing: 11.06.2014
(51) Int. Cl.: G01N 33/68, G01N 33/543

(54) **Method for detecting the development of an infection in humans**

(30) Priority: 11.06.2013 EP 13171428
(71) Applicant: UNIVERSITY OF ZURICH, 8006 Zürich (CH)
(72) Inventor: Graf, Rolf, 8051 Zürich (CH)
(74) Representative: Latscha Schöllhorn Partner AG

(57) **Abstract**

The present invention relates to a reliable method for detecting the development of an infection in humans after a trauma, in particular the development of sepsis, wherein the level of pancreatitis-associated protein (PAP) is determined in serum, and its level is indicative of the development of an infection, in particular of the development of sepsis at early stages of the disease, and is a decision making step for accurate treatment of said sepsis.

## Description

### Field of the Invention

The present invention relates to an *ex-vivo* method for detecting the development of a local or systemic infection in human, in particular for prediction of the development of sepsis and for treatment decision, based on the level of pancreatitis-associated protein (PAP) in body fluids.

### Background of the invention

Systemic responses to severe trauma include a number of parameters affecting innate immunity, inflammatory reactions and cellular activities. Severe trauma patients may have a benign outcome with no infection while others suffer from infections or sepsis. Sepsis is associated with multiple organ failure and a high mortality. Among the most commonly used markers for diagnosis of sepsis are leukocyte counts, C-reactive protein and procalcitonin. The latter are two proteins highly induced after trauma, yet without any known function. In addition, cytokines such as IL-6, IL-8 and IL-18 are employed to monitor patients. However, none of the above mentioned markers serves as a predictive indicator for infections including sepsis, hence treatment may lag behind the onset of sepsis.

In animal pilot experiments, it has been shown that PSP/reg, a protein from a family of pancreatic secretory proteins that includes pancreatic stone protein / regenerating protein (PSP/reg) and the pancreatitis-associated protein (PAP), is induced due to handling stress, even in the absence of pancreatic tissue damage (Graf et al., J. Surg. Res., 2002, 195:136-144*).* Moreover, the PSP/reg level in blood serum was recently proven to be a reliable indicator of systemic sepsis (WO 2009/030456).

PAP, belonging to the same family as PSP/reg, is a 16 kDa secretory protein structurally related to the C-type lectins although classical lectin-related function has not been reported yet. In human, PAP is not expressed in the healthy pancreas, while under condition of acute pancreatitis, it is highly up-regulated and may appear in the serum. PAP expression may be activated in some tissues in an injury- and inflammation-induced manner. In fact, PAP expression is activated in pancreatic acinar cells in response to many injuries such as acute and chronic pancreatitis, hypoxia, toxins, diabetes, and in the transplanted tissue. However, its expression is not restricted to pancreatic tissue, and could be observed in several organs, including the intestine during chronic inflammatory diseases, the brain tissue of Alzheimer patients, and the liver in about 30% of primary hepatocarcinomas.

The regulation of this protein is not due to diet induced secretion like other zymogens. It may appear elevated in other conditions, e.g. during pancreatitis. Thus far, the protein has been studied predominantly in the pancreas. It is also synthesized in PANETH cells of the small intestine (lovanna et al., Am. J. Physiol.,1993: 265; G611-G618). The function of PAP is still debated, but it is generally assumed that it is a secretary stress protein, acting maybe as an anti-inflammatory factor.

### Summary of the invention

The present invention relates to a method of detecting the development of local or systemic infection in humans, in particular for prediction of the development of sepsis, wherein the level of pancreatitis-associated protein (PAP) is determined in an isolated body fluid sample, and its level is indicative of the development of sepsis at early stages of the disease.

### Brief description of the Figures

*Figure 1**: Determination of pancreatitis-associated protein (PAP) in sera of patients after admission to the hospital with a severe trauma.*
   Temporal profile of PAP after a trauma at day 0. All values were combined for each time point. d = days after trauma.
*Figure 2**: Determination of pancreatitis-associated protein (PAP) values in sera of patients after admission to the hospital with a severe trauma.*
   Patients were retrospectively categorized: no infection (SI, n=14), patients with local infection (LI, n=22), patients with sepsis (SE, n=27). PAP values (log₁₀ ng/mL) of the three groups are plotted as box plots with the mean and the 95% confidence interval. d = days after trauma. Statistical analysis was performed using multivariate analysis; p = significance, * = p values for sepsis vs. no infection.

### Detailed description of the invention

The present invention relates to an *ex-vivo* method for detecting the development of a local or systemic infection in humans, in particular for prediction of the development of sepsis, wherein the level of pancreatitis-associated protein (PAP) is determined in an isolated body fluid sample, e.g. serum, and its level is indicative of the development of sepsis at early stages of the disease.

The infection generally occurs consequently to a trauma. The infection can also occur consequently to surgery, in particular to a perforation of the abdomen or the intestine, or consequently to an intubation. The infection can originate from an intra-abdominal region (e.g. peritonitis) or come from the respiratory tract.

As defined herewith, sepsis is a potentially serious medical condition that is characterized by a whole-body inflammatory state (called a systemic inflammatory response syndrome or SIRS) and the presence of a known or suspected infection.

The term "sepsis" as used in the present application summarizes clinical pictures, for which, as a rule, fever, leucocytosis, consciousness changes, a hyperdynamic circulation ("warm shock"), and a hyper-metabolic status, mainly as a consequence of the invasion of the normally sterile tissue by microorganisms, are observed, whereas the positive detection of germs in the blood, which was previously understood to be a characteristic for a sepsis, has become less important for the diagnosis "sepsis". The term "sepsis" as used in this application corresponds to the definition of sepsis as defined in the "Definitions for Sepsis and Organ Failure and Guidelines for the Use of Innovative Therapies in Sepsis" from the ACCP/SCCM consensus conference committee (Bone et al., Chest 1992, 101 ;1644-1655) According to this definition, Sepsis corresponds to a severe inflammatory response syndrome caused by the presence of an infection (either proven or probable).

A systemic infection is one that affects a number of organs and tissues, or affects the body as a whole.

A local infection originates in, and is confined to, one organ system or general area in the body.

As defined herewith, PAP refers to human pancreatitis-associated protein, also called regeneration islet derived or REG III (Orelle et al., J. Clin. Invest., 1992: 90(6); 2284-2291).

As defined herewith, a trauma includes injuries in at least one of six body regions (Head, Face, Chest, Abdomen, Extremities (including Pelvis), External). The severity of a trauma is defined according to the Injury Severity Score (ISS), an anatomical scoring system that provides an overall score for patients with multiple injuries.

In one embodiment, the *ex-vivo* method for detecting the development of a local or systemic infection in humans comprises:
a) providing a body fluid sample from said patient;
b) determining the level of PAP in said body fluid sample;
c) comparing the level of PAP detected in step b) with a reference value;
wherein a higher level of PAP determined in step b), compared to the reference value, is indicative of an infection.

In one aspect of the invention, the reference value is the level of PAP measured in a body fluid sample from a patient without infection.

In another aspect of the invention, the reference value is about 10 ng/ml.

In one embodiment of the invention, the body fluid sample is plasma.

In one embodiment of the invention, the body fluid sample is plasma.

Other body fluids than serum useful for determination of PAP levels are e.g. whole blood, urine, sputum, cerebrospinal fluid, tear fluid, sweat, milk, or extracts from solid tissue or from fecal matter.

The PAP level indicative for development of posttraumatic sepsis is dependent on the body fluid chosen for the determination. For blood serum, this level is of or about 20 ng/ml or more than 20 ng/ml on day 5, 6 or 7, after a trauma that occurred on day 0. Hence, more specifically, the invention relates to a method for detecting the presence of a local or systemic infection, in particular the development of sepsis, wherein the level of PAP is determined in serum, and a level of or about 20 ng/ml or more, is indicative of the development of sepsis. Preferably, the PAP level is of or about 25 ng/ml or more, or more preferably of or about 50 g/ml or more. The PAP level is preferably determined on day 5, 6 and/or 7 after a trauma that occurred on day 0.

In a preferred embodiment of the *ex-vivo* method of the invention, the level of PAP determined in step b) is determined on day 5, 6 or 7 after a trauma that occurred on day 0, and compared to a reference value of or about 10 ng/ml.

Any known method may be used for the determination of the level of PAP in body fluids. Methods considered are e.g. Enzyme-linked immunosorbent assay (ELISA), Radioimmunoassay (RIA),Enzymoimmunoassay (EIA), mass spectrometry, or microarray analysis. Such methods when used for the detection of the development of local or systemic infection, in particular of the detection of the development of sepsis, are a further object of the invention.

A preferred method for the determination of PAP in human body fluids, e.g. serum, is an ELISA. In one embodiment of the invention, the PAP ELISA consists of a sandwich array: conventional microtiter plates are coated with one type of antibody ("first" antibody") directed against PAP. The plates are then blocked and the sample or standard is loaded. After the incubation, a different type of antibody ("second" antibody) against PAP is applied. A third antibody detecting the particular type of the "second" antibody, conjugated with a suitable label, e.g. an enzyme for chromogenic detection, is then added. Finally the plate is developed with a substrate for the label in order to detect and quantify the label, being a measure for the presence and amount of PAP. If the label is an enzyme for chromogenic detection, the substrate is a colour-generating substrate of the conjugated enzyme. The colour reaction is then detected in a microplate reader and compared to standards.

Suitable pairs of antibodies ("first" and "second" antibody) are any combination of guinea pig, rat, mouse, rabbit, goat, chicken, donkey or horse. Preferred are polyclonal antibodies, but it is also possible to use monoclonal antibodies or antibody fragments. Suitable labels are chromogenic labels, i.e. enzymes which can be used to convert a substrate to a detectable coloured or fluorescent compound, spectroscopic labels, e.g. fluorescent labels or labels presenting a visible colour, affinity labels which may be developed by a further compound specific for the label and allowing easy detection and quantification, or any other label used in standard ELISA.

Other preferred methods of PAP detection are radioimmunoassay or competitive immunoassay using a single antibody and chemiluminescence detection on automated commercial analytical robots. Microparticle enhanced fluorescence, fluorescence polarized methodologies, or mass spectrometry may also be used. Detection devices, e.g. microarrays, are useful components as readout systems for PAP.

PAP is a protein that can be cloned from pancreatic mRNA and subcloned into a yeast expression vector. The protein can then be expressed under the control of ADH promoter. A suitable expression medium may comprise methanol to induce and maintain the secretion of PAP. PAP is preferably purified using SP-Sepharose-cellulose by a pH and salt gradient. Such purified PAP is used to prepare standard solutions for comparison with PAP levels in body fluids. Polyclonal antibodies against the protein may be obtained from mice, rats, rabbits, goats, chicken, donkey, horses and guinea pigs or other suitable animals using standard methods.

The invention further relates to a kit of parts for the determination of PAP for diagnosis/prediction of local or systemic infection comprising, for example, apparatus, reagents and standard solutions of PAP. Apparatus considered are e.g. microtiter plates for ELISA, pre-coated ELISA plates, and plate covers. Reagents are those reagents particularly developed and designed for the detection of PAP. Standard solutions of PAP preferably contain PAP synthesized according to the directions hereinbelow. The kit of parts may contain further hardware, such as pipettes, solutions such as buffers, blocking solutions and the like, filters, colour tables and directions for use.

In another aspect, the invention relates to a kit for detecting the development of a local or systemic infection in a patient according to a method of the invention comprising antibodies directed against PAP and reagents.

In a further aspect, the invention relates to a kit for detecting the level of pancreatitis-associated protein (PAP) in a body fluid sample comprising antibodies directed against PAP and reagents.

In a further aspect, the invention relates to a use of a kit according to the invention kit for detecting the development of a local or systemic infection in a patient, notably the development of sepsis for example in a method according to the invention.

In a further aspect, the invention related to a diagnostic method of treatment of sepsis, particularly as a decision-making step where the level of PAP allows clinicians to take accurate treatment actions according to current practice guidelines.

The reason for the increase of PAP in blood serum during early development stages of sepsis is not entirely clear. In rat pilot experiments an increase in PAP synthesis in the absence of pancreatic damage was observed when an intestinal lesion was present, and there was evidence that significant traumatic damage to other organs leads to an increase in blood levels of PAP. For further studies a set of human patients with severe trauma but apparent absence of pancreatic damage was chosen (see Examples). It has also been shown that PAP expression is inducible by cytokines. There is a strong and concerted action of cytokines after trauma. The complexity of the cytokine response, with many different cytokines being released, is not understood. Thus it is likely that PAP expression reacts to cytokines that are raised under condition of systemic stress or trauma. In contrast, other pancreatic enzymes, e.g. amylase and lipase, appear not to be regulated by cytokines, their appearance in the blood being a result of diversion only. The PAP level in blood serum is herewith proven to be a reliable indicator of sepsis. The increase of PAP in blood might imply a specific stress response.

It is shown that like other indicators of inflammation and like PSP/reg, the level of PAP is highly increased in patients during the development of sepsis including before the clinical signs of sepsis are apparent. The detection and quantification of serum PAP is accomplished e.g. by a sandwich ELISA. Normal serum values are around 1 ng/ml. Patients with a severe trauma develop sepsis between day 7 and 10 after the accident causing the trauma. The serum values correlate with the severity of sepsis. They may reach over 200 ng/ml. Before clinical signs of sepsis are available, PAP values start to increase at day 3 to day 5 and reach values above 20 ng/ml. These values allow to predict whether a patient will develop sepsis and hence the need for intensive treatment including costly antibiotic treatment and a stay in the intensive care unit. Compared to commercially available diagnostic assays, the PAP ELISA is a reliable assay to monitor putative septic patients.

### Examples

### Isolation and sub-cloning of PAP

In order to obtain cDNA for the production of PAP specific antibodies, such cDNA is prepared by reverse transcription of pancreatic mRNA using state of the art laboratory methods. A PCR reaction using primers specific for the sequence coding for PAP and selectively amplifying PAP cDNA is performed. The PCR reaction is then repeated with the elongation primer to add a sequence specific for insertion into the *Pichia pastoris* transfection vector. The primer is designed to fuse the coding region of the signal peptide of the alpha-mating factor with a KEX2 site and the coding region of the mature human PAP. Sub-cloning into the *Pichia pastoris* vector is a two-step procedure. First the PAP product is ligated into the pCR2.1 vector (Invitrogen, TAcloning) and the sequence verified. Then the PAP product is cleaved by Xhol/Notl restriction digestion and ligated into transfer vector pPIC9 (Invitrogen). The *Pichia pastoris* strain KM71 (Invitrogen) is transformed and the most productive clone is selected for expansion and production of recombinant protein.

### Large scale expression of protein

Using a single colony, 25 ml of BMG (buffered minimal glycerol, 100 mM potassium phosphate pH 6.0, 1.34% yeast nitrogen base, 4x10-5% biotin, 1% glycerol) is inoculated in a 250 ml baffled flask and grown at 29°C in a shaking incubator (300 rpm) overnight. 10 ml of this culture is used to inoculate 1 liter of BMG in a 3 liter baffled flask and grown at 29°C (300 rpm) overnight. The cells are harvested by centrifugation at 1500-3000xg for 5 minutes at room temperature. Expression is induced by resuspending the cells in 1/5 volume (200 ml) of BMM (buffered minimum methanol, BMG in which glycerol is replaced by 0.5% methanol) in the same baffled flask. 100% methanol is added to achieve a concentration of 0.5% (1 ml) every 24 hrs until optimal time of induction is reached. The cells are harvested by centrifugation at 1500-3000xg at room temperature. The medium supernatant is collected and frozen until purification of the peptide.

The polypeptide is purified from media supernatants. Media supernatants are diluted 1:3 with distilled water. The pH is adjusted to pH 3.5 with HCl. The medium supernatant is then applied to a SP-Sepharose column and eluted by a salt and pH gradient (10 mM LiCl, 50 mM MES, pH 5.3 starting buffer, 2 M LiCl, 50 mM MES, pH 6.3 end buffer). Fractions are collected and analyzed by SDS-gel electrophoresis. The fractions with the highest and purest protein contents are combined and dialyzed against 10 mM HEPES pH 7.5. The sequence of the polypeptide is verified by N-terminal sequencing and the concentration is assessed by amino acid analysis.

### PAP ELISA

In order to determine total PAP, a sandwich ELISA may be used on the basis of a guinea pig antiserum raised against recombinant human PAP and a rabbit antiserum against the same protein. To improve the specificity and sensitivity of the rabbit antibody, IgGs are purified by absorption on a column of protein A beads (HiTrap(R), Pharmacia): A HiTrap(R) column is equilibrated with 200 mM NaH₂PO₄ZNa₂HPO₄ at pH 7. The rabbit antiserum is pH-adjusted with the same buffer solution (final concentration 20 mM) and then loaded onto the column, which is afterwards washed with 100 mM and 10 mM Tris/HCl pH 8 consecutively. The IgG fraction is eluted with 0.1 M citric acid pH 3. The eluted fractions are immediately neutralized with 1 M Tris/HCl pH 8.9. 96-well microtiter plates (Costar EIA plates, flat bottom, high binding) are coated over night at 4°C with guinea pig anti-rat PAP IgG fraction, diluted 1 :500 in TBS (100 µl/well). After a washing step, the plate is blocked with 150 µl 1% BSA/TBS for one hour, which is afterwards replaced by 100 µl of different standard concentrations of recombinant human PAP (0, 0.1 , 0.5, 1.0, 1.5, 2.5, 3.5, or 5.0 ng/ml) or 100 µl samples of diluted sample. Samples and standards are loaded in duplicates and incubated for 1 hr at room temperature. After repeated washing, the plate is incubated for 1 hr with 100 µl rabbit anti- rat PAP IgG, diluted 1:500. Another washing step follows before a 30 min incubation with 100 µl of a commercially available mouse monoclonal anti-rabbit IgG antibody is started (mouse anti-rabbit alkaline phosphatase conjugated, IgG fraction, diluted 1:1000; purchased from Sigma). The plate is then washed again, and a soluble phosphatase substrate, p-nitrophenyl phosphate disodium (Sigma 104(R) tablets), added in alkaline phosphatase buffer (100 mM Tris/HCl pH 9.5, 100 mM NaCl, 0.8 mM MgCl2). After an incubation period of about 20 min optical density (OD) at 405 nm is measured in an MRX microplate reader (Dynatech Laboratories).

All dilutions (except coating antibodies) are prepared in 1% BSA/TBS. All incubations at room temperature are carried out on a rotational ELISA plate shaker (Titramax 100, Heidolph, Bioblock Scientific). All washing steps are performed with TBS/Tween 20 (0.05%, v/v), using an automatic microtiter plate washer (MRW, Dynatech Laboratories).

### Test patients for proof of principle

The study population included 83 injured patients who were admitted to the Division of Trauma Surgery (level I trauma center), University Hospital Zurich, in a time period from January 2002 to September 2003. Inclusion criteria were an injury severity score (ISS) > 16 points, patient age > 16 years, less than four hours between accident and hospital admission, and surveillance on the intensive care unit (ICU) with a survival of more than five days. Patients with a pancreatic injury were excluded. All patients were treated according to the advanced trauma life support (ATLS) guidelines and a standard trauma protocol. In brief, after control of airway, ventilation, and monitoring of cardiovascular functions, life-saving procedures including decompression of body cavities, control of haemorrhage and contamination were carried out. This was followed by radical wound debridement, decompression of compartments, and primary stabilization of major fractures mostly through external fixation ("day one surgery"). Thereafter, patients were transferred to the ICU for restoration of organ functions. Of note, all patients received enteral nutrition within 24 hours after trauma to maintain normal intestinal flora and bowel mucosa. Antibiotics were used, if a septic focus was verified by a positive bacterial culture. In addition, for open fractures standard antibiotics are applied for five days and a single shot of a cephalosporin was given as prophylaxis for osteosynthesis of fractures. Information concerning the blood status of trauma patients, as well as information concerning the levels of standard indicators of inflammation in these patients have already been described *(*Keel et al., Crit. Care Med., 2009, 37(5):1642-1648*).*

### Definition of Systemic Inflammatory Response Syndrome, Local Infection, and Sepsis.

Clinical data including laboratory and vital parameters were collected prospectively over a period of 21 days after the trauma. The occurrences of systemic inflammatory response syndrome (SIRS), local infection, and sepsis were determined by the following parameters. SIRS was defined according to the guidelines of the 1992 American College of Chest Physicians/Society of Critical Care Medicine Consensus Conference. The described criteria were modified inasmuch as they had to be fulfilled for at least three consecutive days to allow the diagnosis of SIRS or sepsis, respectively. SIRS was graded as moderate (two positive criteria) and severe (three or four positive criteria). Patients were categorized post hoc into three groups according to their clinical data and to the described definitions: a) without infection, b) with local infection, and c) with sepsis. Sepsis was diagnosed if all four criteria of SIRS were met for 3 consecutive days in the presence of a septic focus with positive bacterial tissue culture or a positive blood culture. If less than four SIRS criteria were observed over 3 days in the presence of a positive focus, a local infection was diagnosed. Table 1 summarizes demographic data and injury scores at the day of admission.

**Table 1. Demographic data of enrolled patients**

| Parameter | No infection | Local infection | Sepsis |
|---|---|---|---|
| Number | 18 | 32 | 33 |
| Age (yr) | 38.8 ± 15.6 | 37.1 ± 14.4 | 38.5 ± 16.1 |
| Males | 15 (83%) | 20 (62%) | 28 (85%) |
| ISS^{a} (points) | 35.1 ± 9.6 | 32.3 ± 13.8 | 38.9 ± 14.7 |
| GCS^{b} (points) | 9.3 ± 5.2 | 9.7 ± 5.0 | 8.7 ± 5.1 |
| APACHE II^{c} (points) | 14.4 ± 6.5 | 14.9 ± 7.2 | 17.8 ± 7.4 |
| ICU^{d} (days) | 9.6 ± 5.7 | 18.7 ± 11.9 | 30.3 ± 14.9 |

| | | | |
|---|---|---|---|
| Mean ± SD. Values in parentheses are percentages. ^{a} ISS, injury severity score ^{b} GCS, Glasgow coma scale ^{c}APACHE II, acute physiology and chronic health evaluation II ^{d} ICU, intensive care unit. | | | |

### Blood status of trauma patients

Patients are retrospectively assigned to three groups depending on their score: a) no infection, b) local infection and c) sepsis (Table 2). To demonstrate the course of several parameters used to determine the extent of inflammation and injury, blood leukocyte counts and C-reactive protein (CRP) were determined. All patients exhibited a strong reduction of blood leukocytes at day one of hospitalization, irrespective of the severity group. Leukocytes gradually increase to normal with the exception of septic patients which reach a significantly higher leukocyte count of 18x10⁶/L at day 10. Concurrent determination of CRP indicate a gradual increase from low levels at admission to about 150 ng/ml at day three in all groups *(Keel et al., 2009, above).* Although the non-infected group has consistently lower levels than the other groups, there is no obvious pattern that distinguishes the three patient groups. Thus between day 5 and day 7 and between day 14 and 21, septic patients exhibit a higher CRP than non-septic patients, the difference being less than a factor of two.

**Table 2. Injury pattern and posttraumatic course of enrolled patients**

| Parameter | No infection (n = 18) | Local Infection (n = 32) | Sepsis (n = 33) |
|---|---|---|---|
| Head (AIS^{a}, points) | 78% (3.4) | 75% (3.4) | 67% (3.7) |
| Thorax (AIS^{a}; points) | 83% (3.0) | 41% (3.4) | 67.0% (3.5) |
| Abdomen (AIS^{a}; points) | 50.0% (3.9) | 41% (4.1) | 49% (4.3) |
| Extremities (AIS^{a}; points) | 67% (3.0) | 72% (2.5) | 67% (2.6) |
| Pelvis (AIS^{a}; points) | 22% (3.0) | 25% (2.8) | 18% (2.8) |
| Spine (AIS^{a}; points) | 56% (2.9) | 38% (2.8) | 30% (2.3) |
| No SIRS ^{b} | 2 (11%) | - | - |
| SIRS 2 b | 6 (33%) | 4 (12%) | - |
| SIRS 3/4^{b} | 10 (56%) | 28 (88%) | - |
| Sepsis | - | - | 33 (100%) |
| Mortality | 2 (11%) | 2 (6%) | 6 (18%) |

| | | | |
|---|---|---|---|
| ^{a}AIS, abbreviated injury scale ^{b}SIRS, systemic inflammatory response syndrome | | | |

### Determination of standard indicators of inflammation in trauma patients

To determine whether commonly used indicators of inflammation, e.g. IL-6 and procalcitonin (PCT), could distinguish between the three severity groups, blood levels of these proteins were measured during the whole course of the hospital stay. IL-6 immediately increased post-traumatically reaching the highest levels at day 1. During the first two days the three severity groups are different, with the septic group (1200 pg/ml) and the infected group (600 pg/ml) higher than the non-infected group. The statistics do not indicate significance due to the high variability of the data while at day 5-10 there was a difference. Although there is a slight increase during the time of sepsis (350 pg/ml) these levels are low in comparison to the first day of hospitalization *(Keel et al., 2009, above).*

PCT was clearly increased 20-fold in the septic patient group prior to and during sepsis, while the other groups remain at levels around 0.5-2 ng/ml. The maximal increase in PCT in the septic patient group was 25 fold higher than in healthy subjects. The statistics do not indicate significant differences due to the high variation in the sample *(Keel et al., 2009, above).*

### PAP is upregulated post-traumatically

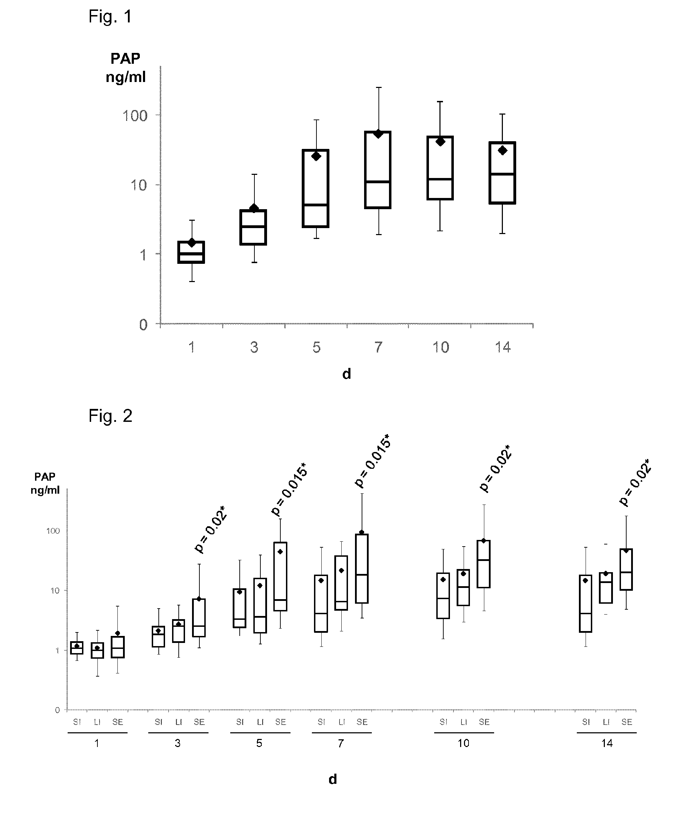

PAP is synthesized predominantly in the pancreas. In response to a local tissue injury it is highly upregulated. Polytrauma causes a release of cytokines that may affect the expression and secretion of PAP. Therefore, it was tested whether PAP is increased in patients with severe trauma that had no pancreatic injury. The combined data of all patients after polytrauma indicate a regular increase of PAP from day one (mean concentration of PAP= 1.4 ng/ml) with a peak at day seven (mean concentration of PAP= 53.2 ng/ml) (Figure 1 and Table 3).

**Table 3. Evolution of the average level of PAP in 83 trauma patients with time after trauma**

| | Days after trauma | | | | | |
|---|---|---|---|---|---|---|
| | Day1 | Day3 | Day5 | Day7 | Day10 | Day14 |
| Mean levels of PAP (ng/ml) | 1.4 | 4.5 | 25.4 | 53.2 | 41.3 | 30.8 |

Thus, PAP is increased after a polytrauma. The data were then analyzed using stratification assigned to patients without infection, patients with local infection and patients with sepsis. PAP values in patients without an infection increased regularly with time after trauma, with a peak at day 7-10. In patients with polytrauma that exhibit a local infection, there is a further increase in serum PAP. Finally, polytraumatic patients exhibiting sepsis show a large increase in serum PAP already visible at day 5 and day 7, when sepsis is not yet clinically confirmed in the majority of the cases (Figure 2).

The increase starts several days before clinical criteria of sepsis are fulfilled. PAP highly correlates with sepsis. At day 7, when patients are not septic yet the average level of PAP in blood increases significantly to over 90 ng/ml and reaches about 6-fold during the time of sepsis (Figure 2).

The early increase of PAP in patients with sepsis can therefore be used as a serum marker to predict sepsis, and therefore allow clinicians to take accurate treatment actions according to current practice guidelines, based on the recommendations of 2008 *(*Dellinger et al, Intensive Care Med, 2008, 34:17-60*),* which are an updated version of the first edition guidelines from 2001 coordinated by the International Sepsis Forum (ISF), and of the second editions from 2004 coordinated by the Society of Critical Care Medicine (SCCM), the European Society of Intensive Care Medicine (ESICM), and ISF (REF).Therefore the specificity, sensitivity and the positive and the negative predictive values are calculated for a cut-off value at 20 ng/ml at day five and seven. The specificity is around 85 percent for a cut-off value of 20 ng/ml, while the sensitivity is around 50% at the same cut-off value. The positive predictive values above are also between 80%-85%, indicating that patients can be identified by this method early on.

## Claims

1. An *ex-vivo* method for detecting the development of a local or systemic infection in a patient comprising determining the level of pancreatitis-associated protein (PAP) in an isolated body fluid sample from said patient.

2. The method according to claim 1, wherein the infection occurs consequently to a trauma.

3. The method according to claim 1 wherein the infection occurs consequently to surgery or to an intubation.

4. The method according to any one of the preceding claims for detecting the development of sepsis.

5. The method according to any one of the preceding claims wherein the body fluid sample is serum.

6. The method according to any one of the preceding claims, comprising:
a) providing a body fluid sample from said patient;
b) determining the level of PAP in said body fluid sample;
c) comparing the level of PAP detected in step b) with a reference value;
wherein a higher level of PAP determined in step b), compared to the reference value, is indicative of an infection.

7. The method according to claim 6, wherein the reference value is the level of PAP measured in a body fluid sample from a patient without infection.

8. The method of claim 7, wherein the reference value is 10 ng/ml.

9. The method of claim 6, wherein the reference value is of 20 ng/ml or, more preferably, 25 ng/ml or, more preferably, 50 ng/ml and is indicative of the development of sepsis.

10. The method of any one of claims 6 to 9 wherein the level of PAP determined in step b) is determined on day 5, 6 or 7 after a trauma that occurred on day 0.

11. The method of any one of claims 1 to 10 wherein the level of PAP is determined by ELISA, RIA, EIA, mass spectrometry, or microarray analysis.

12. The method of claim 11 wherein the level of PAP is determined by a sandwich ELISA, wherein microtiter plates are coated with one type of antibody directed against PAP, the plates are then blocked and the sample or standard is loaded, a second type of antibody against PAP is applied, a third antibody detecting the particular type of the second antibody conjugated with a suitable label is then added, and the label used to quantify the amount of PAP.

13. A kit of parts for detecting the development of a local or systemic infection in a patient according to a method of any one of claims 1 to 12 comprising apparatus, reagents and standard solutions of PAP.

14. A kit for detecting the development of a local or systemic infection in a patient according to a method of any one of claims 1 to 12 comprising antibodies directed against PAP and reagents

15. A kit for detecting the level of pancreatitis-associated protein (PAP) in a body fluid sample comprising antibodies directed against PAP and reagents.
